# EUROPÄISCHE PATENTSCHRIFT

(11) **EP 0 542 188 B1**
(45) Veröffentlichungstag und Bekanntmachung des Hinweises auf die Patenterteilung: **05.02.1997**
(21) Anmeldenummer: 92119186.2
(22) Anmeldetag: 10.11.1992
(51) Int. Cl.: G01N 11/10, G01N 33/04

(54) **Verfahren und Anordnung zur Messung und Überwachung des Verfestigungsverlaufes einer Milchgallerte**
Method and apparatus for measuring and monitoring the solidification of a milk coagulum
Procédé et dispositif de mesure et de contrôle de la rigidification d'un coagulum de lait

(30) Priorität: 13.11.1991 DE 4137274
(43) Veröffentlichungstag der Anmeldung: 19.05.1993
(73) Patentinhaber: SCHWARTE-WERK GmbH, D-21514 Büchen (DE)
(72) Erfinder: Coenen, Johannes, W-4415 Sendenhorst (DE)
(74) Vertreter: Glaeser, Joachim, Dipl.-Ing.

(56) Entgegenhaltungen:
- EP-A- 0 144 443
- US-A- 4 542 645
- JOURNAL OF DAIRY RESEARCH Bd. 49, 1982, GB Seiten 343 - 346 J.E. STORRY ET AL. "Development of coagulum firmness in renneted milk - a two phase process
- MILCHWISSENSCHAFT Bd. 28, Nr. 2, 1973, Seiten 94 - 97 K. STEINSHOLT 'The use of an Instron Universal Testing Instrument in studying the rigidity of milk during coagulation by rennin'
- PATENT ABSTRACTS OF JAPAN vol. 16, no. 136 (P-1333)7. April 1990 & JP-A-32 95 442

## Beschreibung

Die Erfindung betriff ein Verfahren zur Messung und Überwachung des Verfestigungsverlaufes einer Gallerte beim Dicklegungsprozeß von Milch zur Herstellung dickgelegter Milchprodukte gemäß dem Obergriff des Anspruchs 1 sowie eine Anordnung zur Durchführung des Verfahrens gemäß Oberbegriff des Anspruchs 5.

Die Milch verarbeitenden Betriebe, insbesondere Molkereien und Käsereien, unterliegen, ebenso wie andere Bereiche der Nahrungsmittel- und Getränkeindustrie, einer zunehmenden Automatisierung, wobei die Prozeßsteuerung alle Bereiche in wachsendem Maße durchdringt. Hierbei wird allerdings die individuelle Beschaffenheit des Rohstoffes Milch oft nicht genügend berücksichtigt. Der Dicklegungsprozeß von Milch zur Herstellung von dickgelegten Milchprodukten hat zentrale Bedeutung im gesamten Prozeßablauf und ist maßgeblich an der Qualität des Endproduktes beteiligt. Unter Dicklegung von Milch ist im folgenden die Dicklegung von Kesselmilch beim Käsungsprozeß oder die Dicklegung von Milch bei der Herstellung von Quark und Joghurt zu verstehen. Nachfolgend wird zum einen im Zusammenhang mit dem Stand der Technik und zum anderen mit dem nach der Erfindung vorgeschlagenen Verfahren exemplarisch und stellvertretend für technologisch gleichgelagerte Verfahren die Dicklegung von Kesselmilch beim Käsungsprozeß, und hier insbesondere die Problematik bei der Messung und Überwachung des Verfestigungsverlaufes einer Labgallerte im Zuge dieses Prozesses, behandelt. Die im Zusammenhang mit dem Käsungsprozeß angegebenen spezifischen Sachverhalte, Merkmale und Hinweise und die mit dem vorgeschlagenen Verfahren im Zusammenhang mit dem Käsungsprozeß erzielbaren Vorteile sind sinngemäß auch auf andere Herstellverfahren übertragbar, bei denen die Dicklegung von Milch Teil des Herstellungsverfahrens für dickgelegte Milchprodukte ist (beispielsweise Quark- oder Joghurtherstellung).

Der Käsungsprozeß wird oft in starre Zeitabschnitte eingeteilt, die nicht an die Grundvoraussetzungen für eine optimale Käsequalität angepaßt sind. Die Gerinnungsstufe von Milch ist der wichtigste und grundlegende Behandlungsschritt bei der Herstellung von "Käse". Der Zustand der Gerinnung entscheidet wesentlich über die Qualität des sich ergebenden Endproduktes. Einen signifikanten Einfluß auf das Endprodukt Käse hat hierbei insbesondere der optimale Schneidzeitpunkt der Labgallerte. Dieser ist die Grundlage für den weiteren Käsungsverlauf bis hin zum fertigen Endprodukt.

Zum Zeitpunkt des Schneidens soll die Labgallerte die für den weiteren Käsungsprozeß optimale Festigkeit aufweisen, und es soll alles einbaufähige Eiweiß in das dreidimensionale Netzwerk eingebaut sein. Unter diesen Bedingungen weist die ablaufende Molke den niedrigstmöglichen Eiweißgehalt auf, wodurch eine optimale Käseausbeute erreicht wird.

Der Zustand der Gerinnung der Milch und insbesondere der Schneidzeitpunkt werden heute noch, auch in ansonsten hochmodernen Käsereien, nach althergebrachten Methoden (beispielsweise Fingertest) ermittelt, die aber nicht objektiv sein können, weil deren Ergebnisse zu sehr vom individuellen Gefühl und der Erfahrung des Käsers abhängig sind. Eine fortlaufende Überwachung des Verfestigungsverlaufes und eine vollautomatische Bestimmung des optimalen Schneidzeitpunktes ist durch diese konventionelle Methode nicht möglich.

Es wurden andererseits eine große Anzahl von Meßverfahren entwickelt, um die Gerinnung der Milch, d.h. den Verfestigungsverlauf der Gallerte, mit Geräten zu messen (vgl. PROKOPEK, Deutsche Milchwirtschaft 17/1978, Seiten 534ff, (D1)). Einige der bisher entwickelten Meßmethoden bestimmen die Gallertfestigkeit an einem festgelegten Ort, der durch den Aufbau des Gerätes bestimmt ist. Die Gallerte erfährt praktisch an diesem festgelegten Meßort eine Beeinflussung, welche mit Sicherheit einen mehr oder weniger großen Einfluß auf die weitere Entwicklung des Gallertfestigkeitsverlaufes bewirkt. Um die Schädigung der Gallerstruktur zu vermindern, wurde in diesem Zusammenhang vorgeschlagen, die Messungen in Intervallen durchzuführen (W. SCHAR und E. FLÜCKIGER, Separatausdruck aus Lebensmittel-Technologie 15/Nr.5/1982, Abschnitt 2.2, (D2)).

Einige Meßverfahren und die zugehörigen Meßgeräte sind geeignet, die Gallertfestigkeit der eingelabten Milch direkt im Käsefertiger bzw. im Prozeßbehälter zu bestimmen (Schwarte-Ermartic, Schwarte-Käsereimaschinen, Firmenprospekt, Seite 41.4, (D3)). Durch diese Anordnung ist eine Vergleichbarkeit der Gallerten am ehesten sichergestellt. Einige Geräte wiederum arbeiten im Laborbetrieb mit hoher Meßgenauigkeit; sie sind jedoch selbst in modifizierter Form im Käserfertiger bzw. im Prozeßbehälter unter Produktionsbedingungen nicht einsetzbar, da das Meßprinzip eine hohe Empfindlichkeit gegenüber Vibrationen aufweist (Instron Universal-Testgerät, K. STEINSHOLT, Milchwissenschaft 28 (2) 1973, (D4)). So ist beispielsweise aus diesem Grunde das Oszillationsviskosimeter nach Vanderheiden im Käsefertiger bzw. im Prozeßbehälter nicht verwendbar (D.G. BYNUM, Journal of Dairy Science, Vol.65, No.7, 1982, Seiten 1321ff, (D5); Oszillationsviskosimeter (N.I.R.D.), D.S. HATFIELD, wie vor, Vol.34, No.4, 1981, (D6)). Besonders robuste Meßmethoden arbeiten beispielsweise nach dem Prinzip der Widerstandsmessung (Schnittreifemelder nach H. SIEGFRIED, in D1, Abschnitt 2.2). Dabei wird ein kugel- oder kegelförmiger Tauchkörper, der an einem Faden hängt, langsam nach oben durch die gerinnende Milch gezogen. Mit zunehmender Verfestigung wächst die erforderliche Zugkraft, wodurch eine Feder immer mehr gespannt wird. Bei einer bestimmten vorher eingestellten Spannung, die der gewünschten Festigkeit zum Zeitpunkt des Schneidens entspricht, wird ein Kontakt geschlossen, der ein Klingelzeichen auslöst.

Bei einem anderen, ähnlich aufgebauten Gerät ist das Meßelement nicht eine Feder, sondern ein elektrisches Dynamometer, das die Kräfte, die beim Hinausziehen des Tauchkörpers durch die Gallerte auftreten, in elektrische Meßwerte umwandelt. Bei einem vorbestimmten Wert kann auch hier ein Signal ausgelöst werden, das den Schneidzeitpunkt ankündigt (Tauchkörpergerät nach J. Budny, M. Eisele und R. Wasilewski, in D1, Abschnitt 2.2).

Mit den vorgenannten Tauchkörperverfahren läßt sich der Schneidzeitpunkt der Gallerte festlegen, ohne daß dadurch allerdings sichergestellt ist, daß es sich hierbei auch um den optimalen Schneidzeitpunkt handelt, bei dem das gesamte einbaufähige Eiweiß bzw. Casein für den Gallertaufbau verbraucht ist. Andere Methoden, bei denen beispielsweise die zu untersuchende Milch mit Schwingungen beaufschlagt wird und die infolge der Verfestigung veränderliche Fähigkeit zur Übertragung und Absorption dieser Schwingungen gemessen und registriert wird (Gelograph in D2; Oszillationsviskosimeter, in D6), sind zwar eher geeignet, den gesamten Verfestigungsverlauf zu messen, sie können jedoch die Hauptanforderung auch nicht erfüllen, nämlich die Feststellung des Zeitpunktes, bei dem das gesamte einbaufähige Eiweiß bzw. Casein für den Gallertaufbau verbraucht ist.

Aus der DE 34 90 255 C2 ist schließlich ein Verfahren zum Messen der Gerinnung von Milch bekannt, bei dem ein Metalldraht in der Milch angeordnet wird und während der Metalldraht absatzweise oder fortlaufend mit elektrischen Strom versorgt wird, wird die Temperatur des Metalldrahtes über ein gegebenes Zeitintervall gemessen, um den Zustand der Gerinnung der Milch zu bestimmen.

Dieses bekannte Verfahren erlaubt zwar die Messung einer dem Verfestigungsverlauf der Gallerte proportionalen physiklischen Größe, eine Beeinflussung des Gerinnungsprozesses durch die Temperaturerhöhung des Metalldrahtes kann jedoch nicht ausgeschlossen werden. Abgesehen von dieser möglichen Beeinflussung ist der Druckschrift kein Hinweis zu entnehmen, ob bei Anwendung des bekannten Verfahrens Hinweise auf den optimalen Schneidzeitpunkt der Gallerte zu gewinnen sind.

Der vorliegenden Erfindung liegt die Aufgabe zugrunde, den Verfestigungsverlauf einer Gallerte unmittelbar im Prozeßbehälter (insbesonder im Käsefertiger) beim Dicklegungsprozeß von Milch zur Herstellung von dickgelegten Milchprodukten (insbesondere beim Käsungsprozeß) zu messen und zu überwachen, dabei den optimalen Zeitpunkt für die Weiterbehandlung der Gallerte (Schneiden, Rühren oder andere Behandlungsschritte) vollautomatisch zu bestimmen und die Information über den Zeitpunkt im Herstellungsprozeß (insbesondere Käsungsprozeß) als Steuersignal zur Verfügung zu stellen.

Diese Aufgabe wird durch Anwendung der Kennzeichenmerkmale des Anspruchs 1 gelöst. Vorteilhafte Ausgestaltungen des vorgeschlagenen Verfahrens sind Gegenstand der Ansprüche 2 bis 4. Eine Anordnung zur Durchführung des Verfahrens wird durch Anwendung der Kennzeichenmerkmale des Anspruchs 5 verwirklicht. Eine vorteilhafte Ausführungsform der vorgeschlagenen Anordnung ist Gegenstand des Anspruchs 6. Bevorzugte Anwendungen werden in den Ansprüchen 7 bis 9 beansprucht.

Das vorgeschlagene Verfahren bringt ein an sich bekanntes Wegpenetrometer (siehe beispielsweise D1, Seite 535, Abbildungen 3 und 4) zur Anwendung, welches bislang als universelles Härte-, Konsistenz- und Texturprüfgerät für Untersuchungen an zähflüssigen, plastischen und elastischen Stoffen verwendet wurde (im vorliegenden Falle wurde das Penetrometer PNR20 der Firma Sommer & Runge KG, Berlin, zur Anwendung gebracht). Der Prüfkörper dringt dabei mit einer vorgewählten, konstanten Eindringgeschwindigkeit in die Gallerte ein, wobei die dabei ausgeübte Vortriebskraft ständig gemessen und registriert wird. Die Messung erfolgt unter Produktionbedingungen im Prozeßbehälter (beispielsweise Käsefertiger). Durch die geringe Eindringgeschwindigkeit des Prüfkörpers, die in der Größenordnung von 0,1mm/s liegt, bleibt die Struktur der Gallerte weitgehend erhalten.

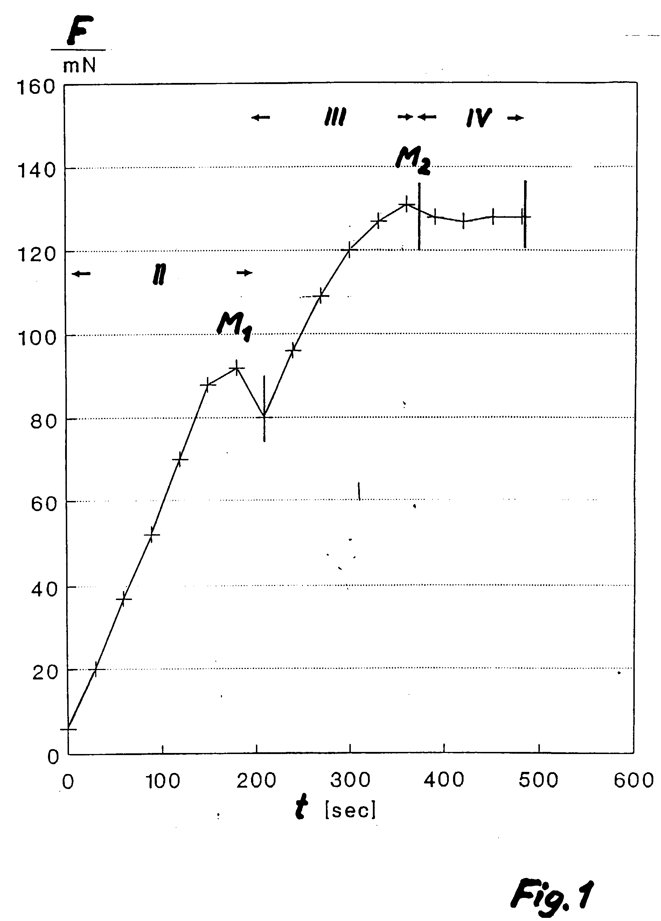

In Figur 1 ist ein den Käsungsprozeß als typisch kennzeichnender Verfestigungsverlauf einer Labgallerte, wie er mit dem vorstehend erwähnten Wegpenetrometer zu gewinnen ist, dargestellt. Auf der Ordinate ist die Vortriebskraft F des Prüfkörpers aufgetragen, die Abszisse stellt die Zeitachse t dar. Durch geeignete Modifikation des handelsüblichen Gerätes kann dessen Hub so weit erhöht werden, daß aus einer als besonders geeignet ermittelten Eindringgeschwindigkeit v eine Zeit für den Gesamthub des Meßgerätes resultiert, die der gesamten Dicklegungszeit der Milch im Käsefertiger bzw. der Milch im Prozeßtank bei technologisch gleichgelagerten Verfahren entspricht. Auf diese Weise kann der Verfestigungsverlauf der Gallerte vom Anfang bis zum Ende verfolgt werden.

Versuchsreihen haben nicht nur ergeben, daß der in Figur 1 dargestellte Verfestigungsverlauf mit einem relativen Maximum M1 und einem weiteren Maximum M2 typisch ist für den in Frage kommenden Gerinnungsprozeß der Milch, wobei dieser markante Verlauf bislang nur im Zusammenhang mit dem vorgeschlagenen Prüfverfahren ermittelt wurde, sondern es wurde nachgewiesen, daß mit dem Erreichen des zweiten Maximums M2 der geringstmögliche Eiweißgehalt in der ablaufenden Molke für die jeweils eingesetzte Milch vorliegt. Wenn nun diese Verhältnisse durch Erreichen des zweiten Maximums vorliegen, dann ist der optimale Schneidzeitpunkt der Gallerte gegeben.

Mit dem vorgeschlagenen Verfahren können der Verfestigungsverlauf und somit das erste relative Maximum und das zweite Maximum für jede Kesselmilch bzw. Milch technologisch gleichgelagerter Verfahren ermittelt und fortlaufend überwacht werden. Bei Erreichen bzw. Überschreiten des zweiten Maximums wird ein Steuersignal für eine Einleitung der Bruchbereitung (Anschneiden oder Rühren oder andere Behandlungsschritte der Gallerte) ausgelöst. Das vorgeschlagene Verfahren basiert auf objektiven Beurteilungskriterien, und es erlaubt die Automatisierung der Dicklegung der Milch und der anschließenden Bruchbereitung.

Um dem Molkereifachmann die Möglichkeit zu geben, den Verfestigungsverlauf zeitgleich zu verfolgen, sehen Ausgestaltungen des vorgeschlagenen Verfahrens vor, daß die Daten des Verfestigungsverlaufes fortlaufend ausgelesen und angezeigt bzw. fortlaufend angezeigt werden.

Eine zusätzliche Sicherheit bei der Beurteilung des optimalen Schneidzeitpunktes der Labgallerte bzw. des Zeitpunktes für das Rühren oder anderer Behandlungsschritte der Gallerte wird im ansonsten automatisierten Ablauf dadurch erreicht, daß, wie dies eine weitere Ausgestaltung des vorgeschlagenen Verfahrens vorsieht, die Weiterverarbeitung des Steuersignales erst nach Freigabe erfolgt. Der die Prozeßsteuerung überwachende Molkereifachmann hat somit die Möglichkeit, den Verfestigungsverlauf in Augenschein zu nehmen und das Steuersignal entweder freizugeben oder aber den Zeitpunkt für die Bruchbereitung noch etwas hinauszuschieben.

Die Anordnung zur Durchführung des vorgeschlagenen Verfahrens geht von einem Prozeßbehälter aus, auf oder an dem die Meßeinrichtung zur Bestimmung der Vortriebskraft des Prüfkörpers angeordnet ist. Dabei hat sich als zweckmäßig herausgestellt, die Meßeinrichtung von der zugeordneten Steuereinrichtung zu trennen. Die Meßeinrichtung weist einen Meßschlitten auf, der seine Vorschubbewegung über eine in den Innenraum des Prozeßbehälters hineingreifende Meßstange auf den Prüfkörper überträgt, und darüber hinaus eine Kraftaufnahmevorrichtung, die die Vortriebskraft mißt. Die Steuereinrichtung ist getrennt vom Prozeßbehälter und somit abgeschirmt von der mehr oder weniger rauhen Produktionsumgebung angeordnet, und sie weist eine Verbindung zu einer Auswerteeinrichtung auf, welche ihrerseits mit einer Prozeßbehälter-Steuereinrichtung und einer Datenausgabeeinrichtung verbunden ist. Mit der vorgeschlagenen Anordnung ergeben sich die besten und zuverlässigsten Meßergebnisse, wenn der Prüfkörper als Doppelkegel oder als Kugel oder als Halbkugel, deren ebene Begrenzungsfläche in Vorschubrichtung zeigt, ausgebildet ist.

Um den gerätetechnischen Aufwand zu reduzieren, sieht eine Ausgestaltung der vorgeschlagenen Anordnung vor, daß die Steuereinrichtung, die Auswerte- und die Datenausgabeeinrichtung eine zentrale Überwachungseinrichtung bilden, die einer Produktionsanlage, bestehend aus mehreren Prozeßbehältern, zugeordnet ist. Jeder Prozeßbehälter ist also lediglich mit einer Meßeinrichtung, die den Meßschlitten, die Meßstange, den Prüfkörper und die Kraftaufnahmevorrichtung beinhaltet, auszurüsten. Für mehrere Meßeinrichtungen sind lediglich eine Steuereinrichtung, eine Auswerteinrichtung und eine Datenausgabeeinrichtung (beispielsweise ein Datensichtgerät und/oder ein Drucker) im Rahmen der Überwachungseinrichtung vorzuhalten. Da mehrere Prozeßbehälter einer Produktionslinie niemals zeitgleich im gleichen Verfahrensschritt betrieben werden, ist eine derartige hardwaremäßige Ausgestaltung der Anordnung kostengünstig und auch technisch zweckmäßig.

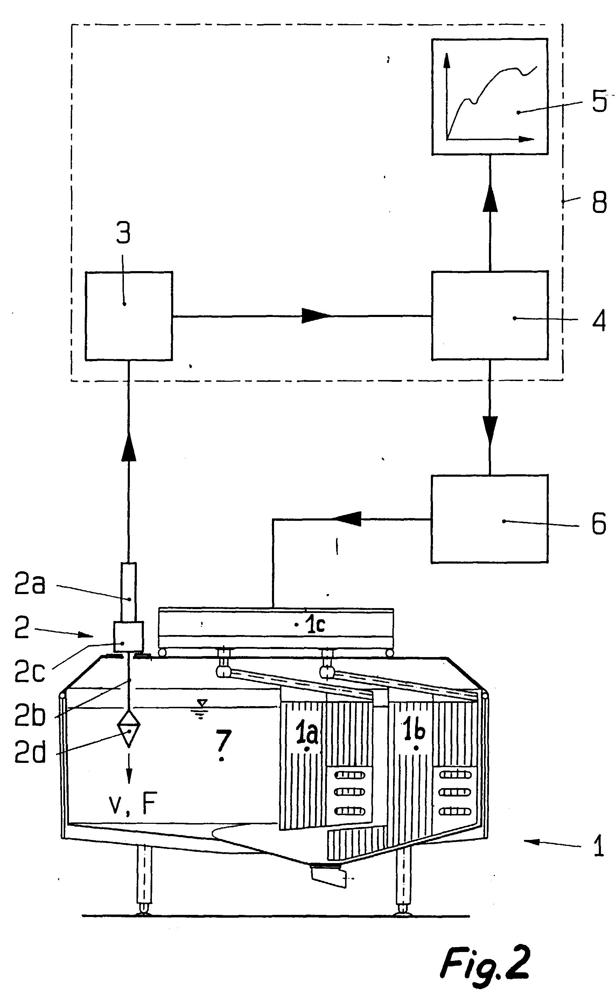

Der typische Verfestigungsverlauf einer Labgallerte beim Käsungsprozeß ist in der vorstehend bereits erwähneten Figur 1 dargestellt; Figur 2 zeigt ein Ausführungsbeispiel der Anordnung zur Durchführung des vorgeschlagenen Verfahrens.

Der Verfestigungsverlauf einer Labgallerte (Figur 1) ist dort in die Abschnitte II, III und IV eingeteilt. Ein vorgeordneter Abschnitt I wurde bei dieser Messung nicht gemessen und daher auch nicht dargestellt, da das handelsüblich zur Verfügung stehende Penetrometer hinsichtlich seines Hubes und damit hinsichtlich der zur Verfügung stehenden Meßzeit begrenzt ist. Der nicht dargestellte Abschnitt I betrifft die Zeit vom Einlaben bis zum Beginn der eigentlichen Messung.

Der Abschnitt II kennzeichnet den Beginn der Messung bis zum ersten Abfall der Meßkurve im Bereich des ersten relativen Maximums M1. In dieser Phase der Messung ist der Prüfkörper gerade unter die Oberfläche der Labgallerte eingetaucht worden und er wurde anschließend mit einer Geschwindigkeit von 0,1 mm/s abgesenkt. Die Gallerte hatte zu Beginn der Messung noch eine Fließfähigkeit, die während des Absenkens in der Umgebung des Prüfkörpers aufgrund ihrer Elastizität zu "Zieh-Effekten" führte. Hieraus resultieren die hohen Meßwerte am Anfang der Meßkurve. Mit zunehmender Festigkeit wurde die Fließgrenze der Gallerte überschritten, und die Verbindung des Prüfkörpers zur Oberfläche der Gallerte riß ab. Hieraus resultiert der relativ starke Abfall der Meßkurve im Bereich des ersten relativen Maximums M1.

In Abschnitt III wird die tatsächliche Gallertfestigkeit gemessen. Die Festigkeit nimmt zu, und zwar bis zu einem Punkt, bei dem das gesamte für den Gallertaufbau zur Verfügung stehende Eiweiß eingebaut ist (zweites Maximum M2).

Abschnitt IV zeigt den Kurvenverlauf im Anschluß an das zweite Maximum M2. Nach Beendigung der Sekundärphase (Phase des Gallertaufbaus) erfolgt die sogenannten Synärese, die mit einer Molkenabgabe verbunden ist. Die austretende Molke führt an der Oberfläche des Prüfkörpers zu "Schmier- oder Gleiteffekten", die einen Abfall der gemessenen Festigkeitswerte und damit den dargestellten abfallenden Meßverlauf zur Folge haben.

In Figur 2 ist in vereinfachter Darstellung ein Prozeßbehälter 1 dargestellt; in diesem Falle handelt es sich um einen Käsefertiger mit Doppelkegelboden und mit zwei Schneid- und Rührflügeln 1a, 1b, die im Behälterinnenraum um zueinander parallele, vertikale Drehachsen angetrieben um- und einander überlappende Arbeitskreise durchlaufen. Mit 1c ist ein Antrieb für die Schneid- und Rührflügel 1a, 1b gekennzeichnet, der mit einer Käsefertiger-Steuereinrichtung 6 (Prozeßbehälter-Steuereinrichtung) verbunden ist. Auf einem nicht näher bezeichneten Deckelteil des Käsefertigers 1 ist eine Meßeinrichtung 2 mit einem Meßschlitten 2a angeordnet, der seine Vorschubbewegung über eine in den Innenraum des Käsefertigers 1 hineingreifende Meßstange 2b auf einen Prüfkörper 2d überträgt. Zur Messung einer am Prüfkörper 2d notwendigen Vortriebskraft F ist eine Kraftaufnahmevorrichtung 2c vorgesehen. Der Meßschlitten 2a verfügt über einen Meßhub, der so bemessen ist, daß bei einer geeigneten Eindringgeschwindigkeit v des Prüfkörpers 2d der gesamte Verfestigungsverlauf einer Gallerte 7 über den gesamten Dicklegungszeitraum sichergestellt ist. Die Daten des Verfestigungsverlaufes (Vortriebskraft F und zugeordnete Zeit t) werden an eine Steuereinrichtung 3 übermittelt und von dieser weiterverarbeitet. Letztere ist mit einer Auswerteeinrichtung 4 verbunden, die den gemessenen Verfestigungsverlauf hinsichtlich des ersten relativen Maximums M1 und des zweiten Maximums M2 untersucht und bei Erreichen des zweiten Maximums M2 ein Steuersignal auslöst.

Über eine mit der Auswerteeinrichtung 4 verbundene Datenausgabeeinrichtung 5 (Drucker, Datensichtgerät) wird der Verfestigungsverlauf fortlaufend ausgelesen und/oder angezeigt. Darüber hinaus ist die Auswerteeinrichtung 4 mit der Käsefertiger-Steuereinrichtung 6 (Prozeßbehälter-Steuereinrichtung) verbunden, so daß das mit Erreichen des zweiten Maximums M2 ausgelöste Steuersignal nach dort weitergeleitet und von dort mittels des Antriebes 1c über die Schneid- und Rührflügel 1a, 1b die Bruchbereitung eingeleitet und durchgeführt werden kann.

Als geeignete Geometrie des Prüfkörpers 2d werden ein Doppelkegel oder eine Kugel oder eine Halbkugel, deren ebene Begrenzungsfläche in Vorschubrichtung zeigt, vorgeschlagen.

Die Steuereinrichtung 3, die Auswerteeinrichtung 4 und die Datenausgabeeinrichtung 5 lassen sich auch in einer zentralen Überwachungseinrichtung 8 zusammenfassen, die einer Käsereianlage (Produktionsanlage), beispielsweise bestehend aus mehreren Käsefertigern (Prozeßbehältern) 1.1, 1.2 bis 1.n, zugeordnet ist.

## Patentansprüche

1. Verfahren zur Messung und Überwachung des Verfestigungsverlaufes einer Gallerte beim Dicklegungsprozeß von Milch zur Herstellung von dickgelegten Milchprodukten, bei dem die zum Vortrieb eines durch die gerinnende Milch bewegten Prüfkörpers erforderliche Kraft zur Beurteilung des Verfestigungszustandes herangezogen wird, wobei der Prüfkörper mit einer vorgewählten, konstanten Eindringgeschwindigkeit in die Gallerte im Prozeßbehälter abgesenkt und die am Prüfkörper erforderliche Vortriebskraft fortlaufend gemessen und gespeichert wird, **dadurch gekennzeichnet, daß**
- aus den Daten des Verfestigungsverlaufes, nämlich aus der Vortriebskraft und der zugeordneten Zeit, die Lage eines ersten relativen Maximums und eines zweiten Maximums ermittelt und
- nach Erreichen des zweiten Maximums ein Steuersignal für eine Einleitung der Bruchbereitung, zum Beispiel Anschneiden, Rühren oder andere Behandlungsschritte der Gallerte, ausgelöst wird.

2. Verfahren nach Anspruch 1, **dadurch gekennzeichnet, daß** die Daten des Verfestigungsverlaufes fortlaufend ausgelesen und angezeigt werden.

3. Verfahren nach Anspruch 1, **dadurch gekennzeichnet, daß** die Daten des Verfestigungsverlaufes fortlaufend angezeigt werden.

4. Verfahren nach einem der Ansprüche 1 bis 3, **dadurch gekennzeichnet, daß** die Weiterverarbeitung des Steuersignales erst nach Freigabe erfolgt.

5. Anordnung zur Durchführung des Verfahrens nach einem der Ansprüche 1 bis 4, bestehend aus einem Prozeßbehälter und einer auf oder an diesem angeordneten Meßeinrichtung zur Bestimmung der Vortriebskraft des Prüfkörpers, wobei die Meßeinrichtung einen Meßschlitten aufweist, der seine Vorschubbewegung über eine in den Innenraum des Prozeßbehälters hineingreifende Meßstange auf den Prüfkörper überträgt, und daß eine Kraftaufnahmevorrichtung vorgesehen ist, die die Vortriebskraft mißt, daß die Meßeinrichtung über eine zugeordnete Steuereinrichtung mit einer Auswerteinrichtung verbunden ist, wobei letztere mit einer Prozeßbehälter-Steuereinrichtung und mit einer Datenausgabeeinrichtung in Verbindung steht, **dadurch gekennzeichnet, daß** der Prüfkörper (2d) als Doppelkegel oder als Kugel oder als Halbkugel, deren ebene Begrenzungsfläche in Vorschubrichtung zeigt, ausgebildet ist, und daß die Auswerteeinrichtung (4) aus den Daten des Verfestigungsverlaufes, nämlich aus der Vortriebskraft und der zugeordneten Zeit, die Lage eines ersten relativen Maximums und eines zweiten Maximums ermittelt und nach Erreichen des zweiten Maximums ein Steuersignal für eine Einleitung der Bruchbereitung auslöst.

6. Anordnung nach Anspruch 5, **dadurch gekennzeichnet, daß** die Steuereinrichtung (3), die Auswerteeinrichtung (4) und die Datenausgabeeinrichtung (5) eine zentrale Überwachungseinrichtung (8) bilden, die einer Produktionsanlage, bestehend aus mehreren Produktionsbehältern (1.1, 1.2 bis 1.n), zugeordnet ist.

7. Verfahren nach einem der Ansprüche 1 bis 6, **gekennzeichnet durch** seine Anwendung auf eine Labgallerte im Käsefertiger beim Käsungsprozeß.

8. Verfahren nach einem der Ansprüche 1 bis 6, **gekennzeichnet durch** seine Anwendung auf eine Säuregallerte im Prozeßtank im Zuge der Quarkherstellung.

9. Verfahren nach einem der Ansprüche 1 bis 6, **gekennzeichnet durch** seine Anwendung auf eine Säuregallerte im Prozeßtank im Zuge der Joghurtherstellung.

## Claims

1. Method for measuring and monitoring the solidification of a coagulum in the thickening process of milk for production of thickened milk products in which the necessary force for propulsion of a body moved through the curdling milk is used for assessment of the solidification state, in which the test body is lowered into the coagulum in the process container at a preselected constant penetration speed, the necessary propulsion force at the test body being continuously measured and stored, characterized in that
- from the data of the solidification, namely the propulsion force and the assigned time, the position of a first relative maximum and a second maximum is determined, and
- after reaching the second maximum, a control signal for starting the production, e.g. cutting, stirring or other treatment steps of the coagulum, is triggered.

2. Method according to Claim 1, characterized in that the data of the solidification are continuously selected and displayed.

3. Method according to Claim 1, characterized in that the data of the solidification are continuously displayed.

4. Method according to one of Claims 1 to 3, characterized in that further treatment of the control signal occurs only after release.

5. Apparatus for performing the method according to one of Claims 1 to 4, consisting of a process container and a measuring device for determination of the propulsion force of the test body mounted on or at the container, with the measuring device providing a measuring carriage transmitting its propulsion movement to the test body over a measuring bar protruding into the interior of the process container, and that a force absorption device measuring the propulsion force is provided, that the measuring device is connected over an assigned control device with an analysis device, the latter being in connection with a control device for the process container and with a data output device, characterized in that the test body (2d) is designed as a double cone or as a sphere or as a hemisphere with its plane surface in propulsion direction, and that the analysis device (4) determines the position of a first relative maximum and a second maximum from the data of the solidification, namely the propulsion force and the assigned time, and after reaching the second maximum produces a control signal for starting the production.

6. Apparatus according to Claim 5, characterized in that the control device (3), the analysis device (4) and the data output device (5) constitute a central monitoring device (8) assigned to a production plant consisting of several production containers (1.1, 1.2 to 1.n).

7. Method according to one of Claims 1 to 6, characterized by its application to a rennin coagulum in the cheese-manufacturer during cheese-making.

8. Method according to one of Claims 1 to 6, characterized in its application to a sour coagulum in the process reservoir during production of curd cheese.

9. Method according to one of Claims 1 to 6, characterized in its application to a sour coagulum in the process reservoir during production of yoghurt.

## Revendications

1. Procédé de mesure et de contrôle de la solidification d'un caillé au cours du processus de caillage de lait lors de la fabrication de produits laitiers caillés, dans lequel la force nécessaire pour faire avancer une sonde de contrôle que l'on déplace dans le lait en cours de coagulation est utilisée pour analyser l'état de solidification, la sonde de contrôle étant plongée à une vitesse de pénétration constante, présélectionnée, dans le caillé, à l'intérieur du réservoir de fabrication et la force de progression requise au niveau de la sonde de contrôle étant mesurée et mémorisée en continu, caractérisé en ce que
- les données relatives à la solidification, c'est-à-dire à la force de progression et au temps correspondant, permettent de calculer la position d'un premier maximum relatif et d'un deuxième maximum et
- un signal de commande est déclenché, une fois le deuxième maximum atteint, pour commencer à briser le caillé, par exemple par découpage, agitation ou d'autres opérations de traitement.

2. Procédé selon la revendication 1, caractérisé en ce que les données relatives à la solidification sont lues et affichées en continu.

3. Procédé selon la revendication 1, caractérisé en ce que les données relatives à la solidification sont affichées en continu.

4. Procédé selon l'une des revendications 1 à 3, caractérisé en ce que le traitement ultérieur du signal de commande ne s'opère qu'après validation.

5. Dispositif pour la mise en oeuvre du procédé selon l'une des revendications 1 à 4, composé d'un réservoir de fabrication et d'un dispositif de mesure monté sur ou contre celui-ci pour déterminer la force de progression de la sonde de contrôle,
le dispositif de mesure comportant un chariot de mesure, qui transmet sa course de progression à la sonde de contrôle par l'intermédiaire d'une tige de mesure pénétrant à l'intérieur du réservoir de fabrication,
un dispositif capteur de force étant prévu pour mesurer la force de progression,
le dispositif de mesure étant relié, par l'intermédiaire d'un dispositif de commande associé, à un dispositif d'analyse, ce dernier étant en liaison avec un dispositif de commande du réservoir de fabrication et avec un dispositif d'édition de données,
caractérisé en ce que la sonde de contrôle (2d) est conformée en double cône, en sphère ou en demi-sphère, dont la surface limite plane est orientée dans la direction de progression,
et en ce que le dispositif d'analyse (4) calcule à partir des données relatives à la solidification, c'est-à-dire de la force de progression et du temps correspondant, la position d'un premier maximum relatif et d'un deuxième maximum, et déclenche un signal de commande pour commencer à briser le caillé, une fois le deuxième maximum atteint.

6. Dispositif selon la revendication 5, caractérisé en ce que le dispositif de commande (3), le dispositif d'analyse (4) et le dispositif d'édition des données (5), constituent un dispositif central de contrôle (8) associé à une unité de production constituée de plusieurs réservoirs de production (1.1, 1.2 à 1.n).

7. Procédé selon l'une des revendications 1 à 4, caractérisé en ce qu'il s'applique à un caillé-présure contenu dans un appareillage de fabrication du fromage, dans un procédé de fabrication de fromage.

8. Procédé selon l'une des revendications 1 à 4, caractérisé en ce qu'il s'applique à un caillé acide contenu dans une cuve de traitement au cours de la fabrication de fromage blanc.

9. Procédé selon l'une des revendication 1 à 4, caractérisé en ce qu'il s'applique à un caillé acide contenu dans une cuve de traitement au cours de la fabrication de yaourt.
